(19) Europäisches Patentamt
European Patent Office
Office européen des brevets

(11) Numéro de publication : **0 053 949**
**B1**

(12) # FASCICULE DE BREVET EUROPÉEN

(45) Date de publication du fascicule du brevet : **28.12.83**

(21) Numéro de dépôt : **81401595.4**

(22) Date de dépôt : **14.10.81**

(51) Int. Cl.³ : **C 07 D495/04**// A61K31/435, C07D211/74 ,(C07D495/04, 333/00, 221/00)

(54) **Procédé nouveau de préparation des tétrahydro-5,6,7,7a-4H-thiéno(3,2-c)pyridinones-2.**

(30) Priorité : **28.11.80 FR 8025275**

(43) Date de publication de la demande : **16.06.82 *Bulletin 82/24***

(45) Mention de la délivrance du brevet : **28.12.83 Bulletin 83/52**

(84) Etats contractants désignés : **AT BE CH DE GB IT LI LU NL SE**

(56) Documents cités :
**FR-A- 2 215 948**
**FR-A- 2 338 703**

(73) Titulaire : **SANOFI, société anonyme**
**40, Avenue George V**
**F-75008 Paris (FR)**

(72) Inventeur : **Maffrand, Jean-Pierre**
**5 Rue du Corps-Franc Pommiès**
**F-31120 Portet-sur-Garonne (FR)**
Inventeur : **Suzuki, Norio**
**942-116 Naganumahara-cho**
**Chiba-Shi Chiba (JP)**
Inventeur : **Matsubayashi, Kiuichi**
**21-22 Yagigaya-cho Funabashi-Shi**
**Chiba (JP)**
Inventeur : **Ashida, Shinichiro**
**1-12 Katemama, 2 Chome Ichikawa-Shi**
**Chiba (JP)**

(74) Mandataire : **Lavoix, Jean**
**c/o Cabinet Lavoix 2, Place D'Estienne D'Orves**
**F-75441 Paris Cedex 09 (FR)**

**EP 0 053 949 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé nouveau de préparation des tétrahydro-5,6,7,7a 4H-thiéno(3,2-c)pyridinones-2

La présente invention est relative à un nouveau procédé de préparation de dérivés de la tétrahydro-5, 6,7,7a 4H-thiéno(3,2-c)pyridinone-2 de formule :

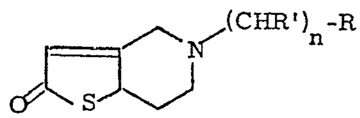

(I)

dans laquelle R représente l'hydrogène ou un radical phényle éventuellement substitué par au moins un atome d'halogène ou un groupe alcoyle inférieur, alcoxy inférieur, nitro, carboxy, alcoxycarbonyle ou cyano ; R' représente l'hydrogène ou un groupe alcoyle inférieur, n est zéro ou un nombre entier de 1 à 4, ainsi que leurs sels d'addition avec des acides minéraux ou organiques.

Ces composés qui présentent des propriétés antiagrégantes plaquettaires et anti-thrombotiques font l'objet de la demande de brevet EP-A-0054442 par la demanderesse. Ils sont par ailleurs inclus dans une formule générale revendiquée dans les brevets français N° 2 215 948 et N° 2 345 150 sous leur forme tautomère suivante :

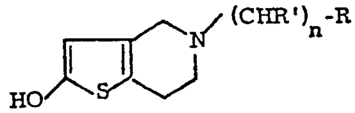

Cependant aucun de ces produits n'est spécifiquement décrit.

On connaît d'après la demande de brevet français N° 2 338 703 un procédé de préparation de dérivés de thiéno-pyridine qui réalise une cyclisation du cycle thiophénique en deux étapes à partir d'un dérivé de pipéridone qui est d'abord condensé avec un dérivé mercaptoacétique, puis cyclisé à l'aide d'une base dans un solvant organique.

L'invention a donc pour objet un procédé de préparation de dérivés de formule I définie précédemment, caractérisé en ce qu'on traite dans un solvant organique un composé de formule suivante :

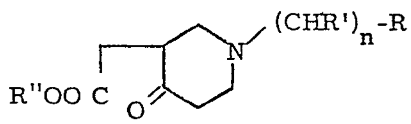

(II)

dans laquelle R, R' et n sont tels que définis ci-dessus et R'' est l'hydrogène ou un radical alcoyle en $C_1$ à $C_4$, par du gaz chlorhydrique et du gaz sulfhydrique.

L'action du gaz chlorhydrique et du gaz sulfhydrique peut être réalisée simultanément par un mélange des deux gaz ou par action séparée et successive de chacun d'entre eux.

Cette réaction est effectuée au sein d'un solvant organique tel qu'un alcanol inférieur, par exemple le méthanol ou l'éthanol, ou un acide carboxylique inférieur, par exemple l'acide acétique ou l'acide propionique, ou dans un mélange de ces solvants. On opère préférablement à des températures comprises entre la température ambiante et la température d'ébullition du solvant.

Les cétoacides ou cétoesters de formule (II) ont été préparés selon un procédé analogue à celui décrit dans la demande de brevet japonais publiée Kokai n° 79 98 771 au nom de Tokkyo Koho et référencée dans Chem. Abs., 1980, *92*, 41773x, pour l'obtention des dérivés de formule II dans lesquels R' = H, R = $C_6H_5$, n = 1 ou 2.

Les exemples suivants illustrent l'invention.

La structure des composés obtenus a été confirmée par microanalyse, spectroscopie infrarouge et résonance magnétique nucléaire.

## Exemple 1

(Chloro-2 benzyl)-5 tétrahydro-5,6,7,7a 4H-thiéno(3,2-c)pyridinone-2

Formule (I) : R = 2-Cl-$C_6H_4$ ; R' = H ; n = 1

a) Préparation du [(chloro-2 benzyl)-1 oxo-4 pipéridine-3] acétate de méthyle.

On ajoute, goutte à goutte, une solution de 100 g (0,355 mole) de [(chloro-2 benzyl)-1 oxo-4 pipéridine-3] carboxylate d'éthyle (J. P. MAFFRAND et D. FREHEL, Bull. Soc. Chim. Fr., 1978, (1-2), II-48) dans 400 cm³ de diméthoxy-1,2 éthane à un mélange de 17,04 g (0,355 mole) d'hydrure de sodium (suspension à 50 %) dans 250 cm³ de diméthoxy-1 2 éthane. On agite à température ambiante pendant 30 minutes puis on ajoute, goutte à goutte, une solution de 59,28 g (0,355 mole) de bromoacétate d'éthyle dans 250 cm³ de diméthoxy-1,2 éthane.

On agite à température ambiante pendant deux heures, filtre le précipité formé, lave celui-ci à l'éther et concentre le filtrat sous vide. Le résidu est repris à l'eau et extrait au chlorure de méthylène. Les extraits organiques sont lavés à l'eau, séchés sur sulfate de sodium, filtrés sur lit de silice et concentrés sous vide.

La résine jaune obtenue (123,2 g) est dissoute dans 850 cm$^3$ d'acide chlorhydrique 6N et la solution est chauffée à reflux, sous atmosphère d'azote, pendant 4 heures. Après refroidissement le milieu est concentré sous vide, additionné d'eau et extrait à l'éther. Les phases aqueuses sont rendues légèrement basiques par addition de soude puis amenées à pH ≃ 4 par addition d'acide acétique et extraites au chlorure de méthylène. Les phases organiques sont lavées à l'eau, séchées sur sulfate de sodium et évaporées à sec. Le produit résineux obtenu (93,8 g) est dissous dans de l'acétone et traité par une solution éthérée de gaz chlorhydrique. Le chlorhydrate de l'acide [(chloro-2 benzyl)-1 oxo-4 pipéridine-3] acétique obtenu est filtré, lavé à l'acétone puis à l'éther et séché sous vide : cristaux blancs, F = décomposition vers 180 °C, rendement : 64 %.

IR(KBr) : $\nu$ CO = 1 728 cm$^{-1}$.

On agite à température ambiante, pendant 3 heures, une solution de 30 g du chlorhydrate précédent dans 300 cm$^3$ de méthanol saturé en gaz chlorhydrique. On évapore sous vide, à une température inférieure à 50 °C, ajoute de l'eau, amène à pH basique par addition de bicarbonate de sodium et extrait au chlorure de méthylène. Les extraits organiques sont lavés à l'eau, séchés sur sulfate de sodium et évaporés à sec. La résine jaune obtenue est utilisée sans autre purification dans l'étape suivante.

IR(film) : $\nu$ CO : 1 720 cm$^{-1}$

RMN($CDCl_3$) : 7,05-7,65 (m, 4H) ; 3,72 (s, 2H) ; 3,62 (s, 3H).

b) (Chloro-2 benzyl)-5 tétrahydro-5,6,7,7a 4H-thiéno(3,2-c) pyridinone-2

On fait simultanément barboter des courants de gaz chlorhydrique et sulfhydrique dans une solution, chauffée à 85 °C, de 4,5 g (0,015 2 mole) de céto-ester précédent dans 45 cm$^3$ d'acide acétique. On concentre sous vide, reprend le résidu à l'eau, amène à pH basique par addition de bicarbonate de sodium et extrait au chlorure de méthylène. Les extraits sont lavés à l'eau, séchés sur sulfate de sodium et évaporés à sec. L'huile jaune obtenue (3,6 g, rendement = 86 %) est transformée en oxalate (F = 170 °C (éthanol)) par addition d'une solution acétonique d'acide oxalique.

IR(KBr) : $\nu$ CO : 1 660 cm$^{-1}$ (large)

Chlorhydrate Hémihydrate : F : décomposition vers 180 °C (précipitation dans l'acétone).

Base : F = 73-74,5 °C (éthanol)

RMN($CDCl_3$) : 7,1-7,6 (m, 4H) ; 6,2 (s, 1H) ; 4,2-4,7 (m, 1H) ; 3,9 (s, 2H) ; 1,5-4,2 (m, 6H).

## Exemples 2 à 9

Les composés suivants ont été préparés selon le même mode opératoire.

Dérivé 2 : Benzyl-5 tétrahydro-5,6,7,7a 4H-thiéno(3,2-c)pyridinone-2
Formule (I) ($R = C_6H_5$ ; $R' = H$ ; $n = 1$)
Maléate : cristaux beiges, F = 132-134 °C (isopropanol)
IR(KBr) : $\nu$ CO : 1 680 cm$^{-1}$
Base : RMN($CDCl_3$) : 7,25 (m, 5H) ; 5,90 (s, 1H) ; 3,60 (s, 2H).

Dérivé 3 : (Chloro-4 benzyl)-5 tétrahydro-5,6,7,7a 4H-thiéno(3,2-c)pyridinone-2
Formule (I) ($R = 4\text{-}Cl\text{-}C_6H_4$ ; $R' = H$ ; $n = 1$)
Maléate : cristaux beiges, F = 158-160 °C (éthanol)
IR(KBr) : $\nu$ CO = 1 680 cm$^{-1}$
Base : RMN($CDCl_3$) : 7,30 (m, 4H) ; 6,0 (s, 1H) ; 3,50 (s, 2H).

Dérivé 4 : (Méthyl-2 benzyl)-5 tétrahydro-5,6,7,7a 4H-thiéno(3,2-c)pyridinone-2
Formule (I) ($R = 2\text{-}CH_3\text{-}C_6H_4\text{-}$ ; $R' = H$ ; $n = 1$)
Oxalate : cristaux beiges, F = 195-197 °C (méthanol)
IR(KBr) : $\nu$ CO = 1 690 cm$^{-1}$
Base : RMN($CDCl_3$) : 7,10 (s, 4H) ; 5,90 (s, 1H) ; 3,55 (s, 2H) ; 2,30 (s, 3H)

Dérivé 5 : [(chloro-2 phényl)-1 éthyl]-5 tétrahydro-5,6,7,7a 4H-thiéno(3,2-c) pyridinone-2
Formule (I) ($R = 2\text{-}Cl\text{-}C_6H_4$ ; $R' = CH_3$ ; $n = 1$)
Chlorhydrate : cristaux jaunes, F = 140-142 °C
IR(KBr) : $\nu$ CO = 1 690 cm$^{-1}$
Base : RMN($CDCl_3$) : 7,30 (m, 4H) ; 6,05 et 5,95 (2s, 1H) ; (2 diastéréoisomères).

Dérivé 6 : [(Chloro-2 phényl)-1 propyl]-5 tétrahydro-5,6,7,7a 4H-thiéno(3,2-c)pyridinone-2
Formule (I) ($R = 2\text{-}Cl\text{-}C_6H_4$ ; $R' = C_2H_5$ ; $n = 1$)
Chlorhydrate : cristaux beiges, F = 124-126 °C
IR(KBr) : $\nu$ CO = 1 690 cm$^{-1}$
Base : RMN($CDCl_3$) : 7,30 (m, 4H) ; 6,05 et 5,90 (2s, 1H) (2 diastéréoisomères).

Dérivé 7 : (Cyano-2 benzyl)-5 tétrahydro-5,6,7,7a 4H-thiéno(3,2-c)pyridinone-2
Formule (I) ($R = 2\text{-}Cl\text{-}C_6H_4$ ; $R' = H$ ; $n = 1$)
Oxalate : cristaux beiges, F = 176-178 °C (acétonitrile)

IR(KBr) : ν CO : 1 700 $cm^{-1}$ : ν CN : 2 210 $cm^{-1}$
Base : RMN($CDCl_3$) : 7,50 (m, 4H) ; 6,00 (s, 1H) ; 3,80 (s, 2H).
Dérivé 8 : (Nitro-2 benzyl)-5 tétrahydro-5,6,7,7a 4H-thiéno(3,2-c)pyridinone-2
Formule (I) (R = 2-$NO_2$-$C_6H_4$ ; R' = H ; n = 1)
Oxalate : cristaux beiges, F = 186-188 °C (isopropanol-éthanol)
IR : ν CO = 1 685 $cm^{-1}$
Base : RMN($CDCl_3$) : 7,50 (m, 4H) ; 5,95 (s, 1H) ; 3,90 (s, 2H).
Dérivé 9 : (Bromo-2 benzyl)-5 tétrahydro-5,6,7,7a 4H-thiéno(3,2-c)pyridinone-2
Formule (I) (R = 2-Br-$C_6H_4$ ; R' = H ; n = 1)
Oxalate : cristaux beiges, F = 151-153 °C (isopropanol)
IR(KBr) : ν CO = 1 690 $cm^{-1}$
Base : ($CDCl_3$) : 7,30 (m, 4H) ; 5,95 (s, 1H) ; 3,75 (s, 2H).

## Revendications

1. Procédé de préparation de dérivés de la tétrahydro-5,6,7,7a 4H-thiéno(3,2-c)pyridinone-2 répondant à la formule générale suivante :

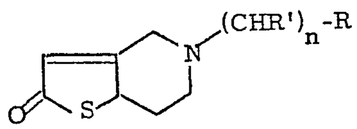

(I)

dans laquelle R représente l'hydrogène ou un radical phényle éventuellement substitué par au moins un atome d'halogène ou un groupe alcoyle inférieur, alcoxy inférieur, nitro, carboxy, alcoxycarbonyle ou cyano ; R' représente l'hydrogène ou un groupe alcoyle inférieur ; n est zéro ou un nombre entier de 1 à 4, ainsi que leurs sels d'addition avec des acides minéraux ou organiques, caractérisé en ce qu'on traite dans un solvant organique un composé de formule suivante :

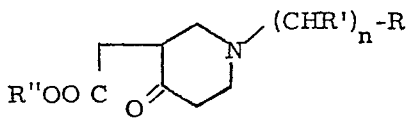

(II)

dans laquelle R,R' et n sont tels que définis ci-dessus et R" est l'hydrogène ou un radical alcoyle en $C_1$ à $C_4$, par du gaz chlorhydrique et du gaz sulfhydrique.

2. Procédé selon la revendication 1, caractérisé en ce que le gaz chlorhydrique et le gaz sulfhydrique sont utilisés en mélange.

3. Procédé selon la revendication 1, caractérisé en ce que le gaz chlorhydrique et le gaz sulfhydrique sont utilisés séparément et successivement.

4. Procédé selon l'une quelconque des revendications précédentes, caractérisé en ce que le solvant organique est un alcanol inférieur ou un acide carboxylique inférieur ou leurs mélanges.

5. Procédé selon la revendication 4, caractérisé en ce que l'alcanol inférieur est le méthanol ou l'éthanol.

6. Procédé selon la revendication 4, caractérisé en ce que l'acide carboxylique inférieur est l'acide acétique ou l'acide propionique.

7. Procédé selon l'une quelconque des revendications précédentes, caractérisé en ce que le traitement par les gaz chlorhydrique et sulfhydrique est réalisé à une température comprise entre la température ambiante et le point d'ébullition du solvant.

## Claims

1. Process for the preparation of 5,6,7,7a-tetrahydro-4H-thieno(3,2-c)pyridin-2-one derivatives of the general formula :

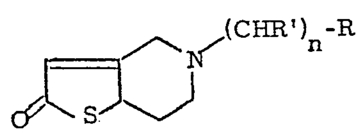

(I)

in which R is a hydrogen atom or a phenyl radical optionally substituted by at least one halogen atom, lower alkyl radical, lower alkoxy radical, nitro group, carboxy group, alkoxycarbonyl radical or cyano

group ; R′ is a hydrogen atom or a lower alkyl radical and n is 0, 1, 2, 3 or 4 ; and of their addition salts with mineral or organic acids, wherein a compound of the general formula :

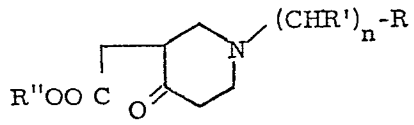

(II)

in which R, R′ and n have the same meanings as above and R″ is a hydrogen atom or an alkyl radical containing up to 4 carbon atoms, is treated in an organic solvent with gaseous hydrogen chloride and gaseous hydrogen sulphide.

2. Process according to claim 1, wherein the gaseous hydrogen chloride and the gaseous hydrogen sulphide are used in the form of a mixture.

3. Process according to claim 1, wherein the gaseous hydrogen chloride and the gaseous hydrogen sulphide are used separately and successively.

4. Process according to any of the preceding claims, wherein the organic solvent used is a lower alkanol or a lower carboxylic acid or a mixture thereof.

5. Process according to claim 4, wherein the lower alkanol used is methanol or ethanol.

6. Process according to claim 4, wherein the lower carboxylic acid used is acetic acid or propionic acid.

7. Process according to any of the preceding claims, wherein the treatment with gaseous hydrogen chloride and gaseous hydrogen sulphide is carried out at a temperature of from ambient temperature to the boiling point of the solvent.

**Ansprüche**

1. Verfahren zur Herstellung von Derivaten von 5,6,7,7a-Tetrahydro-4H-thieno-[3,2]-pyridin(2) on der allgemeinen Formel :

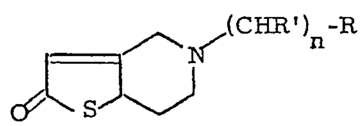

(I)

in der R Wasserstoff oder ein Phenylrest ist, der gegebenenfalls durch wenigstens ein Halogenatom oder einen niederen Alkylrest, einen niederen Alkoxyrest, eine Nitrogruppe, eine Carboxylgruppe, eine Alkoxycarbonylgruppe oder Cyanogruppe substituiert ist, R′ für Wasserstoff oder einen niederen Alkylrest, n für null oder eine ganze Zahl zwischen 1 und 4 steht, sowie ihren Additionssalzen mit anorganischen oder organischen Säuren, dadurch gekennzeichnet, daß man in einem organischen Lösungsmittel eine Verbindung der allgemeinen Formel :

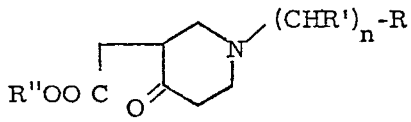

(II)

in der R, R′ und n die vorstehend genannten Bedeutungen haben und R″ Wasserstoff oder ein $C_1$-$C_4$-Alkylrest ist, mit Salzsäuregas und Schwefelsäuregas behandelt.

2. Verfahren nach Anspruch 1, dadurch gekennzeichnet, daß das Salzsäuregas und das Schwefelsäuregas in Mischung verwendet werden.

3. Verfahren nach Anspruch 1, dadurch gekennzeichnet, daß das Salzsäuregas und das Schwefelsäuregas getrennt und aufeinanderfolgend verwendet werden.

4. Verfahren nach irgendeinem der vorstehenden Ansprüche, dadurch gekennzeichnet, daß das organische Lösungsmittel ein niederes Alkanol oder eine niedere Carbonsäure oder ihre Gemische ist.

5. Verfahren nach Anspruch 4, dadurch gekennzeichnet, daß das niedere Alkanol Methanol oder Ethanol ist.

6. Verfahren nach Anspruch 4, dadurch gekennzeichnet, daß die niedere Carbonsäure Essigsäure oder Propionsäure ist.

7. Verfahren nach irgendeinem der vorstehenden Ansprüche, dadurch gekennzeichnet, daß die Behandlung mit den Salzsäure- und Schwefelsäuregasen bei einer Temperatur, die zwischen der Umgebungstemperatur und dem Siedepunkt des Lösungsmittels liegt, durchgeführt wird.